# EUROPEAN PATENT SPECIFICATION

(11) **EP 3 169 391 B1**
(45) Date of publication and mention of the grant of the patent: **26.06.2019**
(21) Application number: 15822711.6
(22) Date of filing: 16.07.2015
(51) Int. Cl.: A61M 16/06, A61M 16/00, A61M 16/08, A61M 16/22

(54) **FACIAL INTERFACE AND HEADGEAR SYSTEM FOR USE WITH VENTILATION AND POSITIVE AIR PRESSURE SYSTEMS**
GESICHTSSCHNITTSTELLEN- UND KOPFBEDECKUNGSSYSTEM ZUR VERWENDUNG MIT BELÜFTUNGS- UND LUFTÜBERDRUCKSYSTEMEN
SYSTÈME DE HARNAIS ET INTERFACE FACIALE À UTILISER AVEC DES SYSTÈMES DE VENTILATION ET À PRESSION EXPIRATOIRE POSITIVE

(30) Priority: 16.07.2014 US 201462025073 P; 16.07.2014 US 201462025077 P; 12.09.2014 US 201462049994 P
(43) Date of publication of application: 24.05.2017
(73) Proprietor: Human Design Medical, LLC, Allston, MA 02134 (US)
(72) Inventor: HARRISON, Donald, Charlestown, MA 02129 (US); GOSLINE, Andrew, Seattle, WA 98115 (US); ARABAGI, Veaceslav, Cambridge, MA 02139 (US); KAPELUS, Aaron, Jamaica Plain, MA 02130 (US)
(74) Representative: Appleyard Lees IP LLP
(86) International application number: PCT/US2015/040737
(87) International publication number: WO 2016/011246

(56) References cited:
- US-A- 2 245 969
- US-A- 4 919 128
- US-A- 5 438 979
- US-A- 5 687 715
- US-A1- 2007 089 749
- US-A1- 2009 188 507
- US-A1- 2010 083 961
- US-A1- 2011 232 649
- US-A1- 2013 008 447
- US-A1- 2013 098 359
- US-A1- 2013 239 301
- US-B2- 8 042 539
- US-B2- 8 636 007

## Description

### BACKGROUND OF THE INVENTION

### 1. Field of the Invention

The present invention relates to medical devices, and, more particularly to mask and headgear portions of air delivery devices that assist with the delivery of gas to the nasal passages of users. These mask and headgear systems and devices may be used with positive airway pressure [PAP] such as continuous positive airway pressure devices [CPAP], automatic positive airway pressure devices [APAP], variable positive airway pressure devices [VPAP], and bi-level positive airway pressure devices [BPAP].

### 2. Description of the Prior Art

Nasal pillows exist to be partially inserted into a user's nare and form a seal with the nare(s), which allows for the user to breathe from the ventilator or PAP device. However, nasal pillows have been known to not necessarily form the best seals for all users, put unnecessary pressure on the nare region when held in place by a mask system, and limited on flexibility. Masks have also tended to be bulky and shift when wearing them at night. Designs are being made to make masks lighter and more secure. US 2 245 969 A is background art and discloses a nasal inhaler. US 5 438 979 A is background art and discloses a nasal cannula support.

A need therefore exists for a nasal pillow that is interchangeable with a mask system, which is flexible and adaptable to a user's nare and facial profile, and reduces pressure applied on the nare region while in use. A need also exists for an adjustable mask and headgear system that conforms to a user's head and facial features while being comfortable and securely attaching the nasal pillows to a user's nares.

### SUMMARY OF THE INVENTION

The invention is defined by the appended claims.

In accordance with an aspect of the present invention, there is provided a mask and headgear assembly as set out in accompanying claim 1, e.g. for use with ventilation and positive air pressure systems. The assembly may be configured to provide a portion of continuous airway pressure to a user's airways. The assembly includes a core having an inlet connector for receiving a supply of pressurized gas from a delivery tube, the core including a right arm and a left arm both extending from the core, each arm forming an associated air pathway through each respective arm, wherein each arm includes an aperture. The assembly further includes a nasal pillow assembly configured to connect to each of the arms over the respective apertures. In this manner each nasal pillow assembly is configured to communicate the supply of pressurized gas from the air pathway through each nasal pillow assembly and to a user's nostrils.

A headgear interface is also provided which is located about a distal end of each of the right and left arms, the headgear interface being configured to be attached to a headgear assembly.

In some embodiments the right and left arms can be offset with respect to one another so as to be non-coaxial, or in other words angled with respect to one another. In yet other embodiments the nasal pillow assembly includes a nasal pillow rotatable about a nasal pillow axis.

In some embodiments the headgear interface provided at each distal end of the left and right arms can include a deformable sidepiece configured to attach to its respective arm. This deformable sidepiece can be configured to attach to the arm at various angular positions with respect to the axis of its respective right or left arm. In some embodiments the deformable sidepiece as a planar member which is configured to be selectively deformed out of plane so as to conform about the facial contours of a user, for example, to hold a shape corresponding to the curvature of the user's cheeks. It will be appreciated that this deformable sidepiece represents a potentially uncomfortable situation wherein the deformable sidepiece could be pressed into the user's face. As such, a malleable cover, such as fabric or neoprene can be provided and configured to encompass the deformable sidepiece.

In some embodiments the nasal pillow assembly can further include an attachment sleeve configured to engage with each of the right and left arms respectively and encompass the associated aperture. The attachment sleeve can thus be configured to provide rotation of each pillow assembly about its respective arm without obstructing flow through the respective aperture. In some embodiments the attachment sleeve includes a radial hose connection for interfacing with its respective nasal pillow. This radial hose connection can be configured to allow for axial adjustable along the radial hose.

In yet other embodiments the attachment sleeve can be provided with one or more washout vents. Alternatively, washout vents can be provided at distal ends of the right and left arms, or about the core, or in any combination of the same.

In some embodiments the nasal pillows can formed in the shape of a cone, the cone having an elliptical cross section. In this manner as the pillows are rotated about a central pillow axis, or about the axis of the radial hose the relative orientation of each pillow can be adjusted so as to match the nostrils or nares of the user.

In some embodiments the headgear can include a plurality of adjustable straps so as to be adjustable to provide a desired retention force or a desired sealing force as well as be customizable so as to match the specific contours of the user's head. In some embodiments one strap can be configured to extend over a crown of the user's head, and in other embodiments a strap can be configured to extend behind a rear portion of the user's head, or both.

The deformable sidepiece may attach to each arm using an interference interconnector comprising a male connection and a female connection located selectively about either the deformable sidepiece or the interference interconnector.

In some embodiments the inlet connector can includes a swivel connector so as to provide a certain degree of flexibility with respect to an air supply hose and the mask frame provided about the user's face, for example if the user shifts while sleeping.

In some embodiments the core can be provided with a heat moisture exchange (HME) located within the central portion. Alternatively, the HME can be provided within the air supply hose, or within the right or left arms

Also disclosed is a method of providing a pressurized stream of air using the device described above is contemplated. The method can include various steps, in varying combinations including: providing a supply of pressurized gas to a delivery tube; receiving the supply of pressurized gas at an inlet of a core; selecting a pair of properly sized nasal pillows from a plurality of various nasal pillows, each nasal pillow having a pillow aperture formed at a top end; affixing the pair of nasal pillows to the core over the respective apertures of each arm such that the air pathway extends through the pillow aperture of each pillow; and positioning the nasal pillows such that the air pathway extends to a user's respiratory system through the nasal pillows through the user's nares.

The method can also include the steps of: affixing a headgear assembly to distal ends of both the right and left arms; and rotating the nasal pillows such that the elliptical cross section coincides with the user's particular nare shape, wherein each of the nasal pillows has an elliptical axial cross section.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 illustrates a perspective view of a facial interface and headgear system for use with ventilation and positive air pressure systems;
FIG. 2 illustrates a front exploded view of the facial interface and headgear system for use with ventilation and positive air pressure systems of Fig. 1;
FIG. 3 illustrates a core or mask frame structure for use with the facial interface and headgear system for use with ventilation and positive air pressure systems of FIGs. 1-2;
FIG. 4 illustrates an exploded view of the core or mask frame structure of FIG. 3 illustrating a swivel adapter and heat moisture exchange component.
FIG. 5 illustrates an exemplary headgear system attached to the core or mask frame structure of FIG. 3;
FIGs. 6A-E illustrate various exemplary nasal pillows and configurations for use with the ventilation and positive air pressure systems of FIGs. 1-2;
FIG. 7 illustrates a top view of the ventilation and positive air pressure systems of FIGs. 1-2;
FIG. 8 illustrates an exemplary embodiment of a potential headgear connection interface for use with the ventilation and positive air pressure systems of FIGs. 1-2;
FIG. 9 illustrates another exemplary embodiment of a potential headgear connection interface for use with the ventilation and positive air pressure systems of FIGs. 1-2;
FIG. 10 illustrates a fitting for the potential headgear connection interface of FIG. 9;
FIG. 11 illustrates another alternative fitting for the potential headgear connection interface of FIG. 9;
FIG. 12 illustrates a perspective view of an assembly procedure using the headgear connection interface of FIG. 9;
FIG. 13 illustrates a perspective view of an assembly procedure of yet another exemplary embodiment of a potential headgear connection interface for use with the ventilation and positive air pressure systems of FIGs. 1-2;
FIG. 14 illustrates an alternative perspective view of the assembly procedure of the embodiment of FIG. 13;
FIG. 15 illustrates a perspective view of a user wearing yet another exemplary embodiment of a potential headgear connection interface for use with the ventilation and positive air pressure systems of FIGs. 1-2;
FIG. 16 illustrates a perspective view of the assembled exemplary embodiment of a potential headgear connection interface of FIG. 15;
FIG. 17 illustrates a perspective exploded view of the exemplary embodiment of a potential headgear connection interface of FIG. 15;
FIG. 18 illustrates a perspective view of an assembly procedure of the exemplary embodiment of a potential headgear connection interface of FIG. 15;
FIG. 19 illustrates a perspective view of another portion of the assembly procedure of the exemplary embodiment of a potential headgear connection interface of FIG. 15;
FIG. 20 illustrates a perspective view of a user wearing yet another exemplary embodiment of a potential headgear connection interface for use with the ventilation and positive air pressure systems of FIGs. 1-2;
FIG. 21 illustrates a perspective exploded view of a yet another exemplary embodiment of a potential headgear connection interface for use with the ventilation and positive air pressure systems of FIGs. 1-2;
FIG. 22 illustrates a perspective view of yet another partially assembled exemplary embodiment of a potential headgear connection interface for use with the ventilation and positive air pressure systems of FIGs. 1-2; and
FIGs. 23A-C illustrate exploded side and front views, respectively, of an alternative core or mask frame assembly for use with the ventilation and positive air pressure systems of FIGs. 1-2.
FIG. 24 illustrates variable core or mask frame with pivoting arms.

### DETAILED DESCRIPTION OF THE PREFERRED EMBODIMENT

**To** provide an overall understanding of the systems, devices, and methods described herein, certain illustrative embodiments will be described. Although the embodiments and features described herein are frequently described for use in connection with CPAP apparatuses, systems, and methods, it will be understood that all the components, mechanisms, systems, methods, and other features outlined below may be combined with one another in any suitable manner and may be adapted and applied to other PAP apparatuses, systems, and methods, including, but not limited to, APAP, VPAP, and BPAP apparatuses, ventilators, systems, and methods.

The present application seeks to provide a solution to the aforementioned problems by creating an adjustable, comfortable, mask assembly system that has interchangeable components, light-weight, and adaptable to individual users.

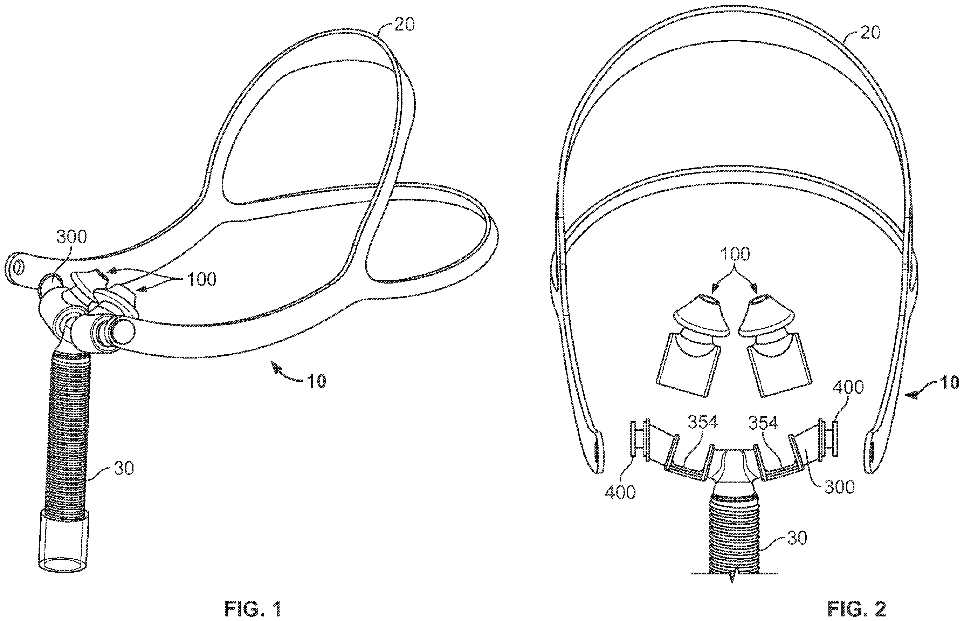

Figs. 1-2, and 7 illustrate various views of a positive airway pressure assembly 10 configured to aid in supplying a stream of positive pressure air to the airways of a patient wearing the assembly 10. The assembly includes a mask frame 300 having a pair of nasal pillow assemblies 100 attached thereto. The mask frame 300 receives a stream of pressurized air from a blower (not shown), which can be attached to the mask frame 30 by means of a supply hose 30. The air then travels through the mask frame 30 through apertures 354 and through the associated pillow assemblies 100 to provide air into the nostrils or nares of the user wearing the positive airway pressure assembly 10.

The positive airway pressure assembly 10 can optionally include a headgear system 20 configured to provide a sealing force between the individual pillow assemblies 100 and the nostrils of the user. In certain cases the headgear system 20 can also provide a positioning force between the mask frame 300 and the maxilla of the user or patient, for example on the portion of the face between the upper lip and below the nose. It will be appreciated that the headgear assembly 20 can be formed of a resilient material, or be adjustable through various means so as to conform to the individual user's contours which, understandably, vary between various users. Further, the headgear assembly 20 can also be configured to affix to distal ends of the mask frame 300 and can be configured to provide a certain degree of rotational adjustment between the mask frame 300 and the headgear 20.

As shown in various figures, headgear 20 may be comprised of multiple straps, such as one configured to go over the top portion of a user's head, and second strap going generally about the back portion of a user's head. Either strap can have an adjustment mechanism, no adjustment mechanism, formed of resilient material, inflexible or formed in a variety of configurations including having a cover or sleeve formed over a portion of the straps or no cover or sleeve.

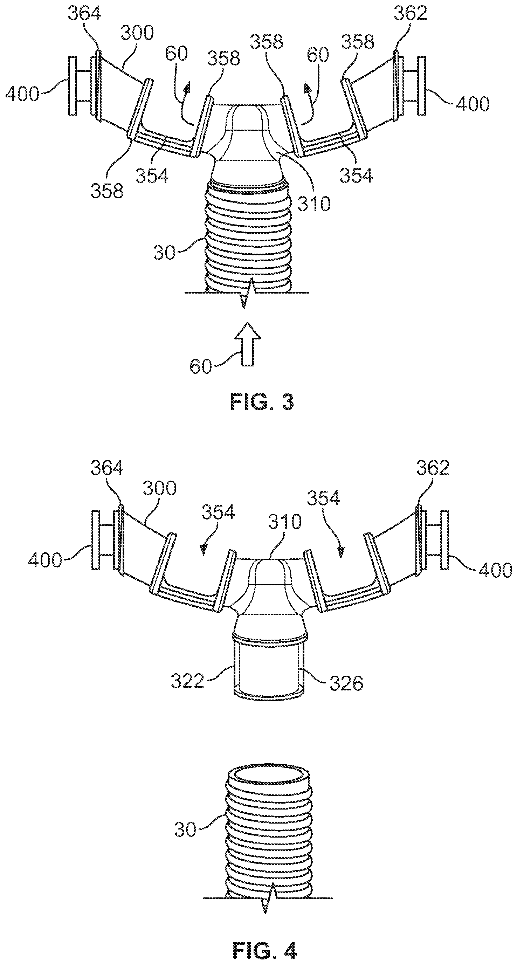

FIGs. 3-5 illustrate various aspects of the mask frame 300. It will be appreciated that air supply travels as shown by pathway arrows 60 through the tube, through a central portion of the mask frame 300 and exits apertures 354. The apertures can have a pair of lips or shoulders 358 upon or about which the pillow assembly 100 from FIGs. 1-2 can rest and seal. The mask frame 300 can have a central portion 310 and left and right arms extending therefrom, 362 and 364 respectively. Each of the right and left arms can be provided with a headgear connection interface 400 about their respective distal ends. The headgear connection interface allows for variation in the types of connectors used for connecting the headgear (not shown here).

In some embodiments, the right and left arms can be provided as co-axial, i.e. straight with respect to each other, so as to reduce fabrication complexity and cost. Alternatively, and as shown herein the right and left arms can be angled with respect to one another so as to better conform in shape to the front of the user's face, which understandably typically has a curved profile.

In addition the mask frame or core 300 can be provided with an inlet connector 322 about the central portion. The inlet connector can be configured to swivel coaxially with the air supply hose 30. In addition the core or mask frame 300 can be provided with a heat moisture exchange (HME) component within the core 326 about the inlet connector 322. The HME 326 can also be provided in alternative locations as well as in multiples, for example a pair of HME 326 units could be provided within the nasal pillow assemblies or more proximal the apertures 354.

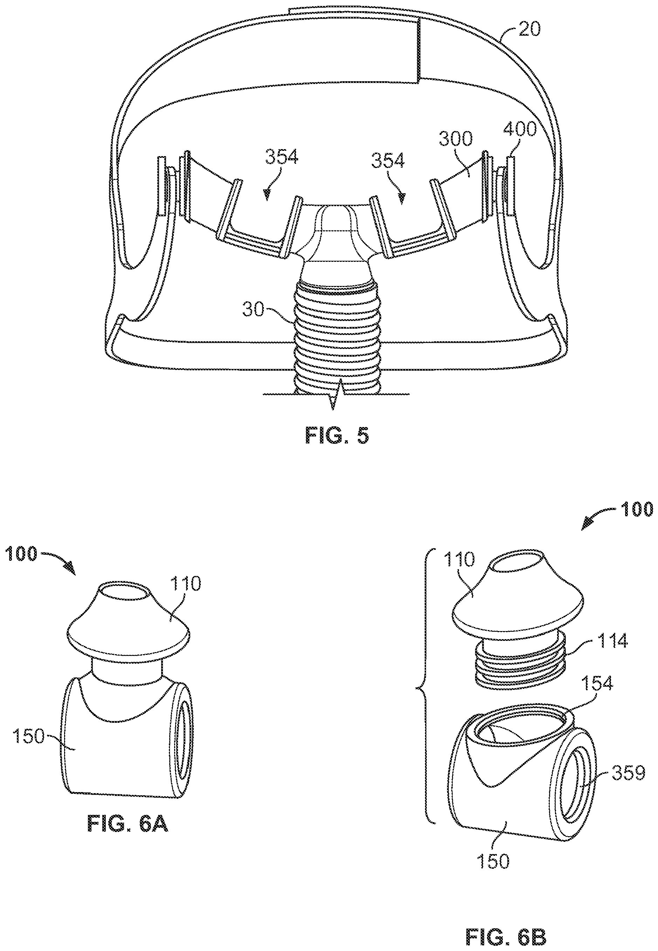

In particular, FIG 5 illustrates how the headgear can be affixed to the core or mask frame 300 through the use of one embodiment of a headgear connection interface 400. This particular embodiment illustrates a swivel connection which allows the headgear to rotate with respect to the distal ends of the mask frame 300.

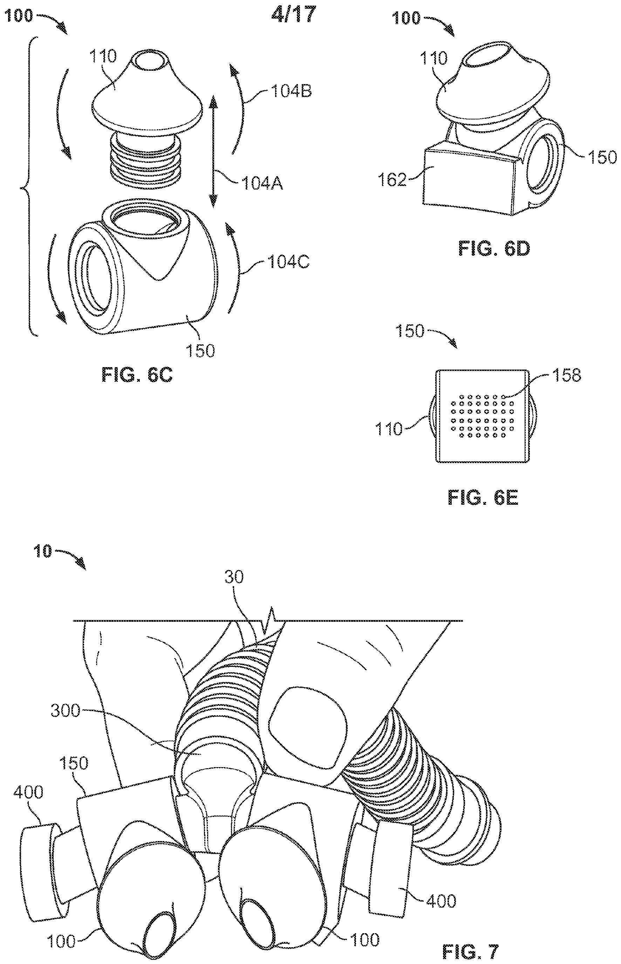

FIGs. 6A-E illustrate various views of a nasal pillow assembly 100 for use with the nasal mask frame as shown in FIGs. 3-5. The nasal pillow assembly 100 can include a nasal pillow 110 and attachment sleeves 150. The attachment sleeves 150 in this embodiment are configured to slide over the mask frame 300 and seal over apertures 354 by having an inner shoulder 359 which abuts against and slidingly seals against the shoulders 358 as shown in FIG. 3. In this manner, the air delivered to the mask frame can be redirected through the pillow assembly 100 and into the user's nares. The attachment sleeve 150 can be provided with an attachment portion 154 for receiving the pillow 110. The attachment portion 154 can be provided with a series of ribs or channels configured to interface with a plurality of annular ribs 114 and/or channels provided on an annular tube (or stem) forming an attachment portion of each pillow 110.

In particular FIG. 6D illustrates an air conform bladder 162 which can be formed as part of the attachment sleeve 150. The air conform bladder 162 can be formed of a malleable or flexible material, and have a hollow cavity defined thereby which receives pressurized gas from the interior of the attachment sleeve 150 when attached to the mask frame (not shown here). In this manner, as the pressure rises or is increased when the system is on, the air conform bladder becomes partially inflated and acts similar to a balloon. The air conform bladder 162 can then rest against the maxilla and provide an air cushioned interface between the mask and the user's face. In some embodiments, the air conform bladder is formed directly on the core frame, as part of the nasal pillows devoid of an attachment sleeve, or a part of the attachment sleeve itself that can form in part the nasal pillow assembly.

The meshing or integration of the annular ribs 114 with the channels or ribs 154 provided in the attachment sleeve allows for incremental adjustment of the relative height or radial positioning of the nasal pillow 110 with respect to the attachment sleeve 150, and thereby the mask frame or core, by changing which ribs are meshed with which respective channel. In this manner each nasal pillow can translate axially with respect to a pillow axis thus providing a first degree of freedom 104A. Additionally, the ribs and channels can slide with respect to one another when twisted about the pillow axis providing a second degree of freedom 104B which is rotational about a central axis of each pillow. Finally, the interior shoulder 359 can also slide with respect to its relative exterior shoulder of the mask frame 358 as shown in FIG. 3. so as to allow the sleeve, and the associated pillow to rotate about the axis of the right or left arm thus providing a third degree of freedom 104C. This sealing lip 359 allows for the attachment sleeve 150 to rotate about the mask along the mask frame axis thus providing a third degree of freedom 104C. Additional flexibility in the system can come from the nasal pillow itself. For example, the base portion of the nasal pillow, which functions like a trampoline or pivoting spring allows for the head or conical portion of the nasal portion to tilt or pivot about the stem or annular tube. This is made possible by varying the thickness or durometer of the base portion with respect to the head or conical portion and the stem or annular tube.

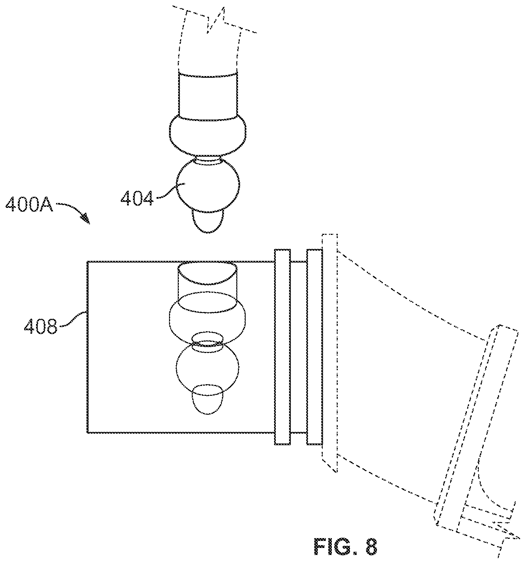

FIG. 8 illustrates another embodiment of the headgear connector 400A which utilizes a contoured barb 404 and a corresponding barb receiver 408. The barb can have a plurality of shapes including semi-spherical shapes as shown, or any other conceivable geometric shape with a correspondingly shaped receiver. In this embodiment the receiver is configured to be deformable or resilient so as to expand to initially accept the barb 404 when press therein. After the barb 404 is pressed into the receiver, an interference fit is formed and the barb will resist, to a certain degree, being pulled from the receiver 408.

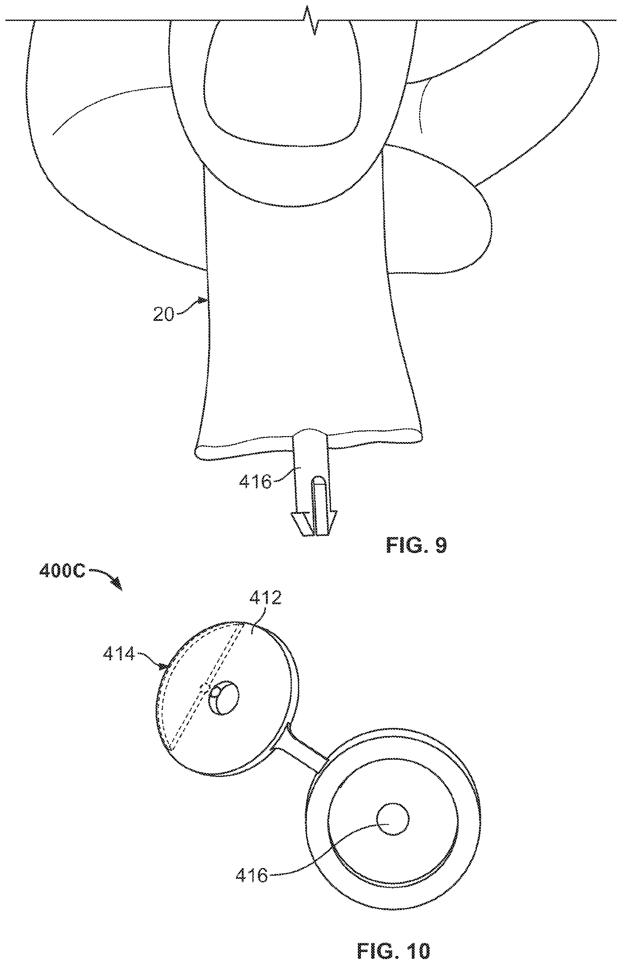

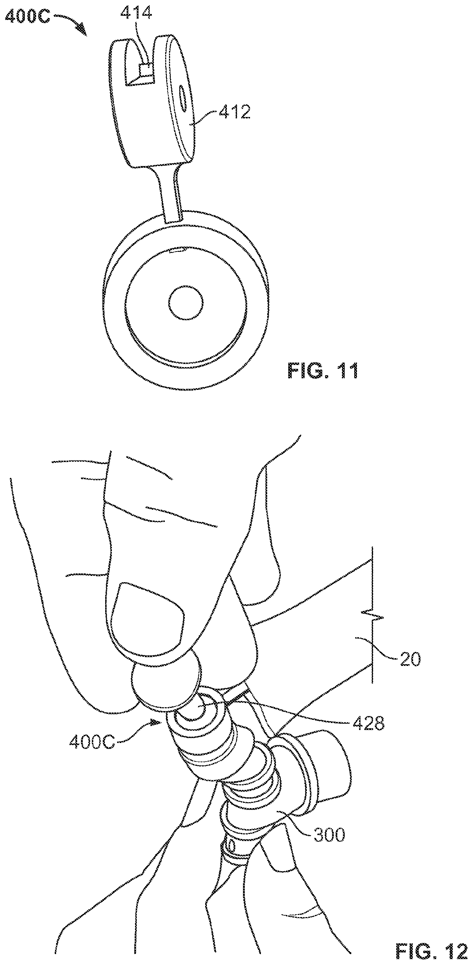

FIGs. 9-12 illustrate yet another embodiment of a headgear connector 400C which utilizes a connector 412 which has two ends, one for attaching to the distal end of the mask frame or core 300, and the other for interfacing with the headgear 20. The headgear interfacing end is provided with an aperture 414 configured to receive a clip barb 416. The core end of the connector 412 has another corresponding aperture 416 through which a plug 428 can be provided so as to affix the connector 412 to the core 300. The two ends of the connector can be configured to rotate with respect to one another, as illustrated between FIGs. 10 and 11, so as to provide additional comfort to the user and allow the strap of the headgear to rest naturally with respect to the distal ends of the mask frame.

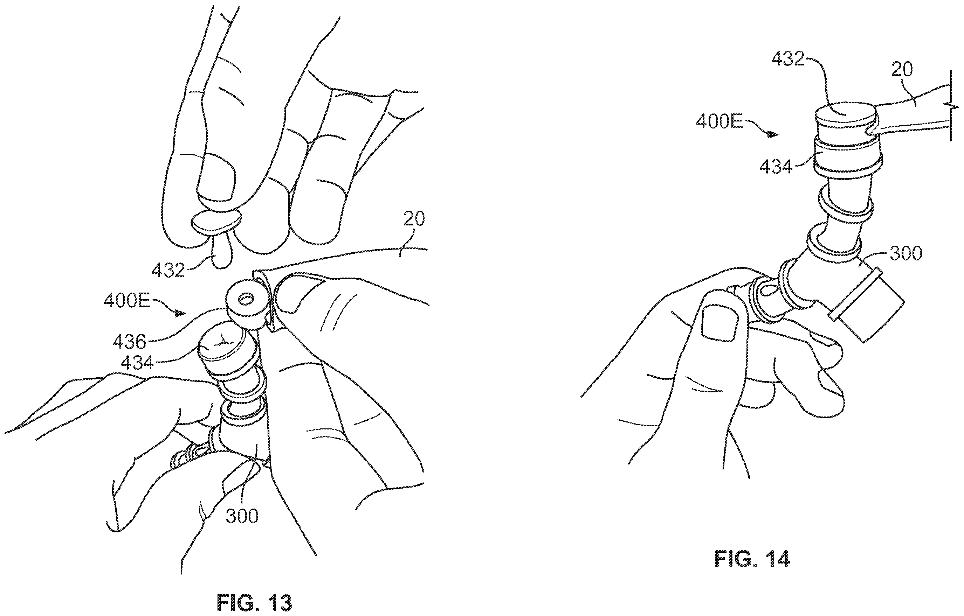

FIGs. 13-14 illustrate yet another embodiment of a headgear connection interface 400E in which a strap of the headgear 20 is provided with a simple annular washer end 436. A plug 432 can then be provided the annular washer end 436 and have an interference fit with a corresponding plug adapter end 434 provided about the distal ends of the mask frame 300.

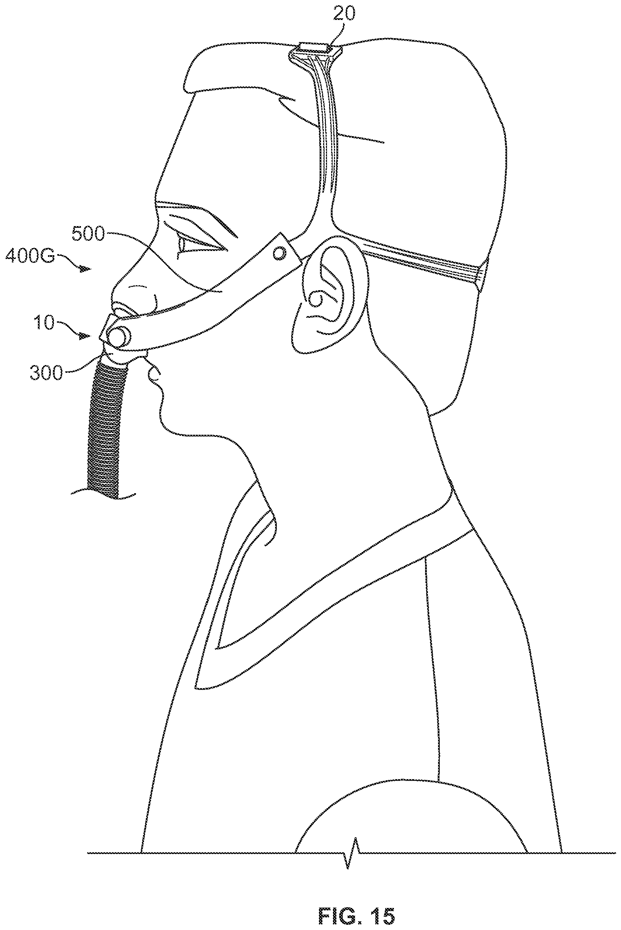

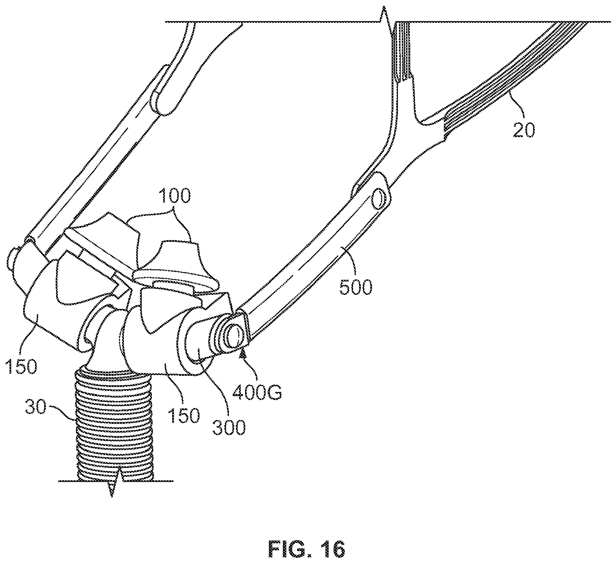

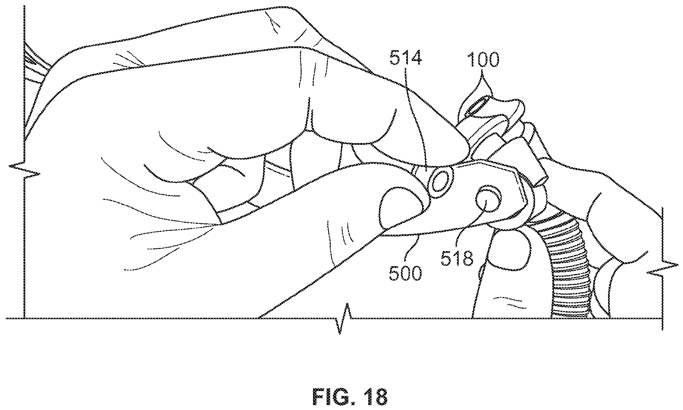

FIGs. 15-19 illustrate various views of yet another embodiment of a headgear connection interface 400G in which a strap of the headgear 20 is provided with a deformable side piece 500 provided between the headgear 20 and the mask frame 300. The deformable sidepiece 500 can attach to each arm using an interference interconnector comprising a male connection 518 and a female connector 514 as well as attached to the headgear 20 by means of a male connector 522 and female aperture 524. It will be appreciated that the relative male of female connectors or apertures can be located selectively about either the deformable sidepiece or the interference interconnector. As shown, the deformable sidepiece 500 can be configured to attach to the each respective arm at various angular positions, or in other words rotate with respect to the mask frame 300. Additionally, the deformable sidepiece 500 can be provided initially as a planar member, which can then be selectively deformed out of plane so as to conform about the facial contours of a user. In this manner the deformable side piece can be shaped so as to follow the contours of the user's cheeks without touching them, or alternatively touch the cheeks but equally distribute any pressure applied thereto.

It should be understood that of the various connectors described herein, some versions are configured to have the headgear connect to the mask frame in a fixed connection (non-rotating), some allow for free rotation connection (no interference or stops), and some have interference mechanisms to selectively rotate or be positioned angularly about the mask frame.

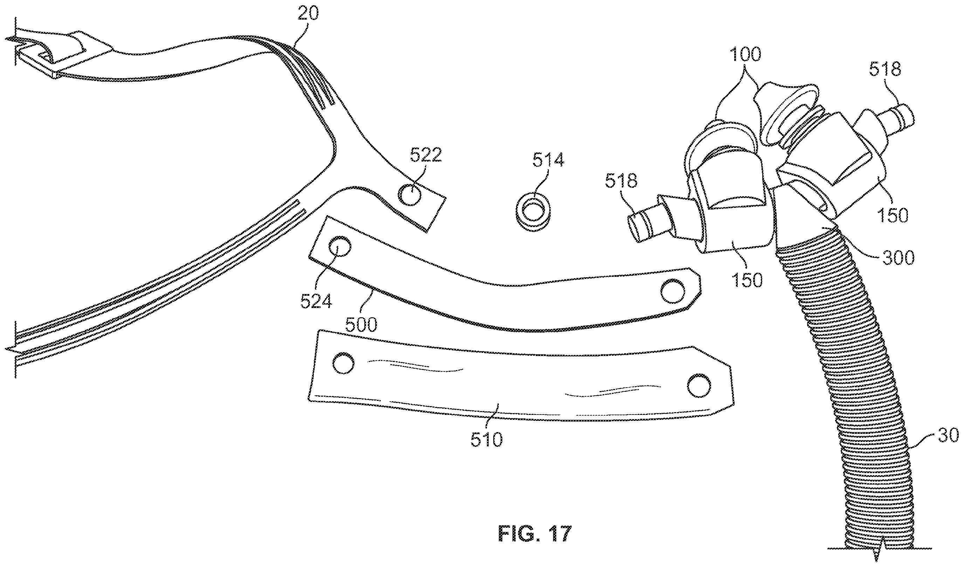

In one instance the deformable sidepiece is formed of a shape retaining plastic. This plastic can have a general deformation characteristic along a single plane while maintaining some rigidity in a second plane. Other types of deformable plastic can be deformed along multiple planes. In one embodiment the cross-section of the deformable sidepiece is rectangular. The curvature of the deformable sidepiece along a particular plane (see Figs.15 and 17) can be preset or formed to transfer the force of the head gear system around certain features of the user's face. Since user's faces have three-dimensional features the deformable sidepiece can then conform to the remaining features of the user's face. Thus, allowing a customizable headgear system that maintains a balance between rigidity and flexibility, while being conformable to a user's unique facial features.

It will be further appreciated that the deformable sidepiece 500 might cause a certain degree of discomfort to a user. As such, a malleable sleeve 510 can be provided which encompasses the deformable sidepiece 500. The malleable sleeve can be formed of fabric, silicone, or other comfort increasing material having any number of desired attributes, such as heat transfer rate, elasticity, softness, etc.

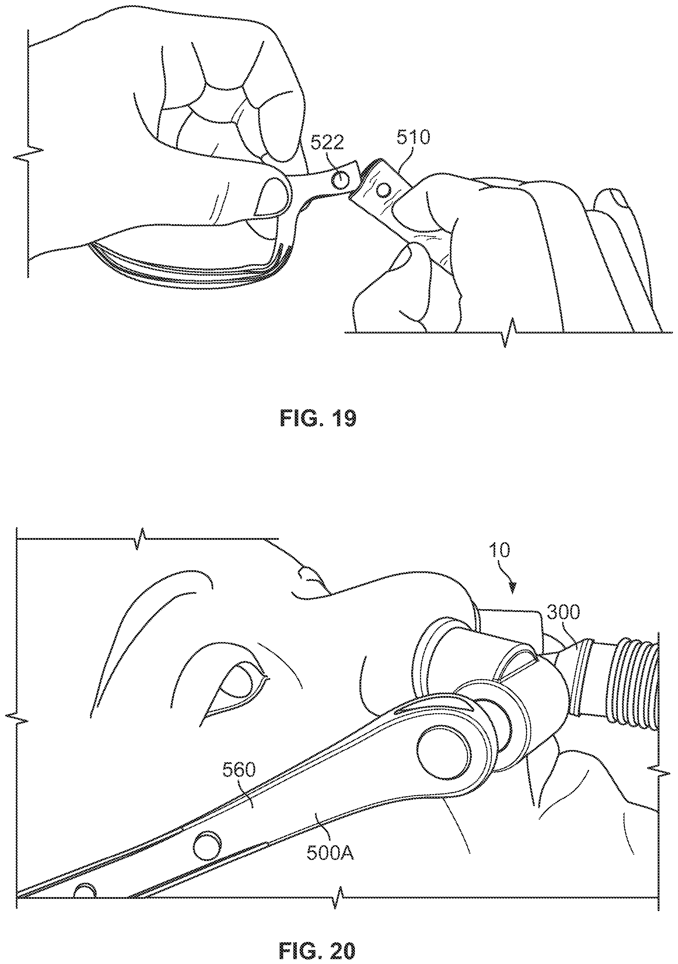

FIG. 20 illustrates a deformable sidepiece 500A which has a silicone shell 560 having a malleable shape retaining core.

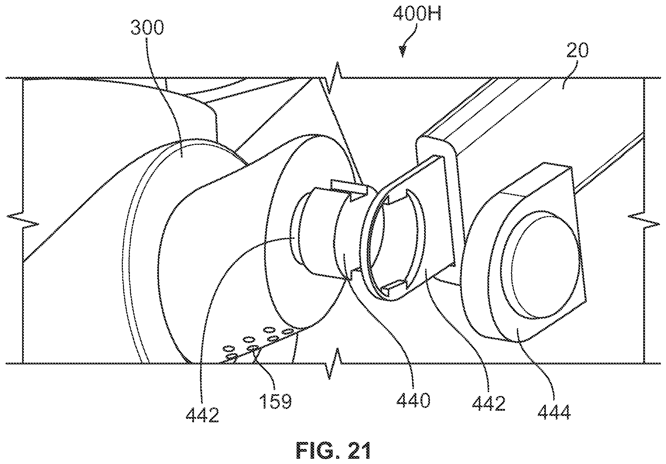

FIG 21 illustrates yet another headgear connection interface 400H which includes a keyed post 440 located about a distal end of the mask frame 300 and keyed opening 442 which slid through the keys to an inner portion 442 with a smaller diameter which allows free rotation. The assembly can only be separated when angularly positioned correctly so as to align the keys. It will be appreciated that the keys should be provided out of phase from each other in normal angular positions between the mask frame 300 and the headgear 20 while being worn. In order to ensure that the keyed components do not separate unintentionally, a cap 444 can be provided which prevents unintentional separation.

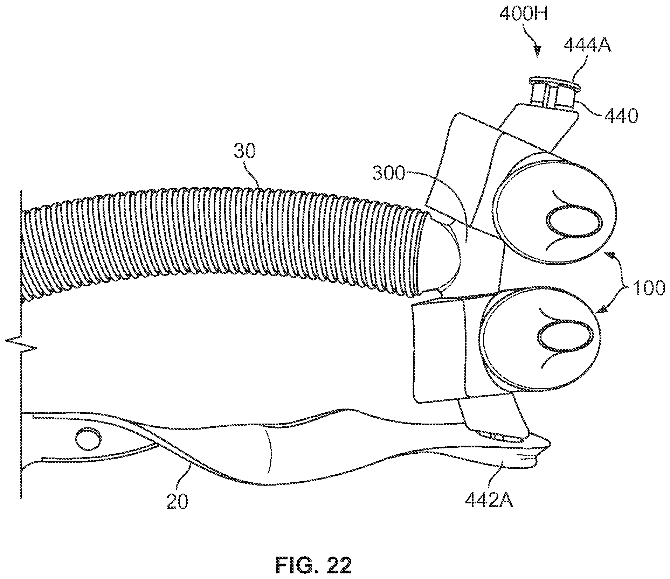

FIG. 22 illustrates another keyed embodiment, similar to that of FIG. 21. having an alternative strap portion 442A, which covers the hardware, i.e. the keyed post 440 and the associated connector inside the strap 442A, so as to improve comfort and reduce the likelihood of catching the mask on something while shifting during sleep and thus tearing the mask off the user's face. This embodiment utilizes a similar plug 444A to cover the connection from the outside of the strap 442A and thus prevent premature decoupling or catching.

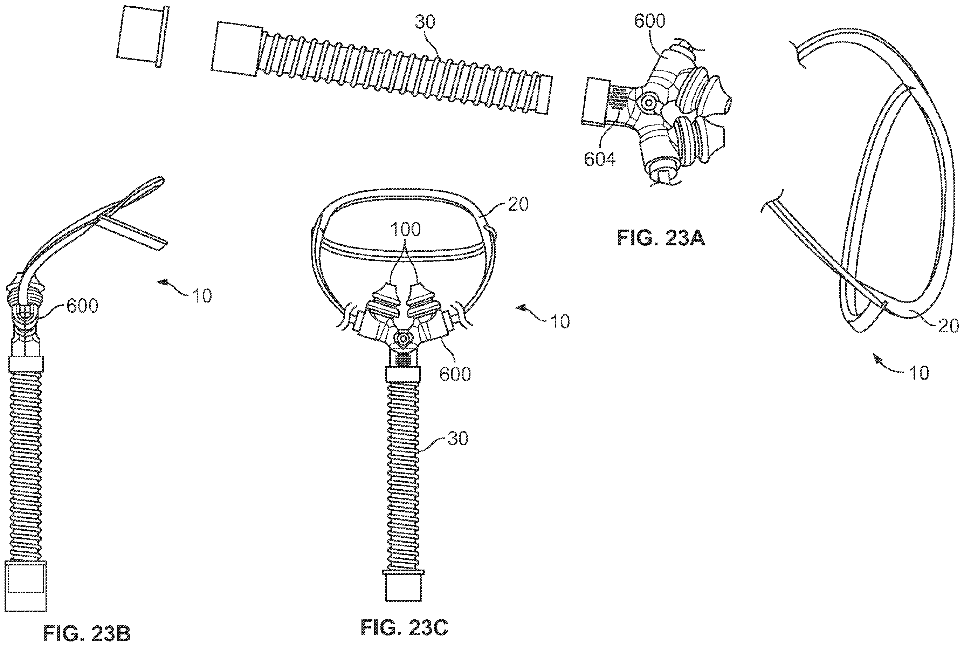

FIGs. 23A-C illustrate an alternative embodiment of a mask frame 600. This mask frame is more rigid and instead of interfacing with the nasal pillow assembly 100 using a rotatable sleeve, the arms of mask frame 600 are rigid and do not provide rotation of the pillow assemblies 100 about the respective arm portions. This embodiment provides increased stability for headgear attachment and facial placement purposes. In this embodiment the nasal pillows are still permitted to rotate about the pillow's central axis, wherein the pillows can have an elliptical cross section.

In this embodiment a plurality of washout vents 604 can be provided in a central portion of the mask frame 600. Additionally, the headgear 20 can be attached to the mask frame 600 using any of the previously discussed headgear attachment interfaces.

FIG. 6E, 21 and 23A all show various placements of CO₂ washout vents. Being at a bottom portion of the pillow assembly 100, on the attachment sleeve 150 as shown by 158 in FIG. 6E, at the ends of the right or left arms, as shown by 159 in FIG. 21, and on the mask frame at a central portion as shown by 604 in FIG 23A. It will be appreciated that any one of these placements either alone or in any combination is within the scope of the present invention. The CO₂ washout vents may be comprised of a material that has silicone knife coated across it. In other embodiments the CO₂ vent is a plurality of holes that have been formed therein.

It is contemplated that the wall thickness and/or durometer of the nasal pillow portion can be varied. In one exemplary embodiment the flat underside portion which connects the bell like top of the nasal pillow to the tube portion may have either a thinner wall portion then the flared bell like portion and tube portion or may have a lower durometer value. This thinner wall or lower durometer value allows the tube connected to the flat underside to collapse into the bell like portion when pressure is exerted on the bell like portion. When the nasal pillows are formed of the silica material or silken like material the nasal pillow returns to its original state when no pressures being exerted on it. Again this allows for the flared bell like portion to pay that about the tube portion when being inserted into the nasal region. The collapse ability again helps reduce pressure exerted onto the nasal region while at the same time helping to find an optimal position that forms a good seal between the nasal pillow and each of the nostrils.

It will be appreciated that in certain embodiments the headgear can cause a direct tightening of the pillows into the nostrils of the user, thus having a direct correlation to a sealing force. In yet other embodiments, for example, when providing an air conform bladder, as discussed with reference to FIG. 7, the force applied by the headgear can be partially directed through the air conform bladder and into the maxilla to provide a primarily a positioning force, where the sealing force can be adjusted by changing the relative placement of the mask frame on the face, which is held by the positioning force. In yet additional embodiments, the nasal pillows can be caused to enter into, and hold their relative position by the elastic properties of the pillows being exerted onto the inner walls of the user's nostrils or nares without the use of headgear altogether.

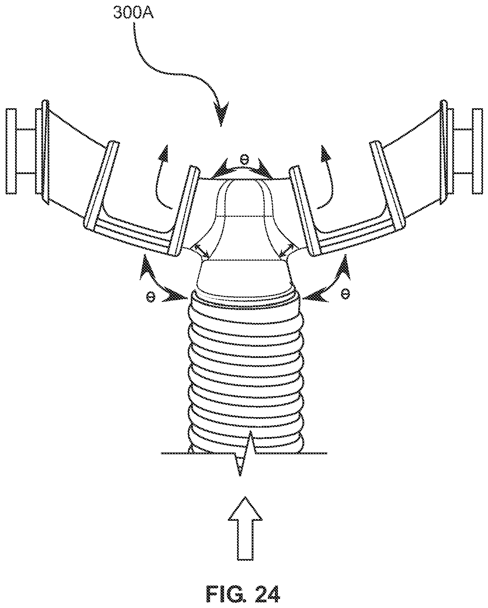

Fig. 24 illustrates another alternative core or mask frame 300A where the right and left arms are arranged to pivot or rotate about the center of the core. In some versions the right and left arms can form a 180 degree angle between each other, making the core look more like "T" shape, each arm can then be repositioned to form a "Y" shape. The angles between each arm can range from several degrees to greater than 180 degrees. However, most users will have the arms angled somewhere less than 180 degrees. This additional degree of freedom presented by this alternative core 300A can also work with the attachment sleeves, rotatable nasal pillows as described above for a customizable fit.

In some versions the rotation of the arms is a constant and consistent motion, which can be enabled by a pressure sliding fit between the pivoting arm and the core. In other versions discrete angled positions are enabled by each arm locking into a groove or channel or other distinct locking mechanism. Some of the rotation mechanisms can function similar to the locking and rotation features of the headgear interface assembly.

## Claims

1. A mask and an headgear assembly, the mask comprising:
a core (300) having an inlet connector (322) for receiving a supply of pressurized gas from a delivery tube, the core including a right arm (364) and a left arm (362) each extending from the core in opposing lateral directions, each arm forming an associated air pathway through each respective arm, wherein each arm includes an aperture provided through a sidewall thereof;
a pair of nasal pillow assemblies (100), each nasal pillow assembly having at least a nasal pillow (110) rotatably connected over the aperture of a respective right arm and left arm, each nasal pillow assembly being configured to communicate the supply of pressurized gas from the air pathway through each nasal pillow assembly and to a user's nostrils;
a headgear interface (400) located about a distal end of each arm, the headgear interface being configured to be attached to the headgear assembly.

2. The mask and headgear assembly of claim 1, wherein each headgear interface further comprises a selectively deformable sidepiece (500) configured to attach to its respective arm.

3. The mask and headgear assembly of claim 2, wherein the selectively deformable sidepiece can be configured to attach to the arm at various angular positions.

4. The mask and headgear assembly of claim 2, wherein the selectively deformable sidepiece is planar and configured to be selectively deformed out of plane so as to conform about the facial contours of a user.

5. The mask and headgear assembly of claim 2, further comprising:
a malleable sleeve (510) configured to encompass the selectively deformable sidepiece.

6. The mask and headgear assembly of claim 1, further comprising an attachment sleeve (150) configured to engage with each of the right and left arms respectively and encompass the associated aperture, the attachment sleeve configured to provide rotation of each nasal pillow assembly about each respective arm so as to maintain flow through the respective aperture.

7. The mask and headgear assembly of claim 6, wherein the attachment sleeve includes a radial hose connection for interfacing with a respective nasal pillow, wherein each nasal pillow is axially adjustable along the radial hose.

8. The mask and headgear assembly of claim 6, wherein each attachment sleeve is provided with one or more washout vents (158).

9. The mask and headgear assembly of claim 1, wherein an angle formed between the right and left arms is less than 180 degrees.

10. The mask and headgear assembly of claim 1, wherein the core further comprises:
one or more washout vents (640) located about a central portion.

11. The mask and headgear assembly of claim 1, wherein the core further comprises:
one or more washout vents (159) located about the distal ends of both the right and left arms.

12. The mask and headgear assembly of claim 1, wherein the core further comprises:
a heat moisture exchange device (326) located about a central portion.

13. The mask and headgear assembly of claim 2, further comprising a flexible cover disposed over a portion of at least one of the selectively deformable sidepieces of the headgear assembly.

14. The mask and headgear assembly of claim 1, wherein the nasal pillow assembly further comprises a pair of attachment sleeves (150) disposed between each arm of the core and the nasal pillows, and wherein the attachment sleeves rotate at least partially about each arm while maintaining the communication of flow of pressurized gas from the air pathway through to nasal pillows.

15. The mask and headgear assembly of claim 1, wherein the right and left arms pivot about the core.

## Patentansprüche

1. Maske und Kopfbedeckungsanordnung, wobei die Maske Folgendes umfasst:
ein Kernelement (300), das ein Einlassverbindungsstück (322) zum Empfangen einer Zufuhr von mit Druck beaufschlagtem Gas von einem Zuleitungsrohr aufweist, wobei das Kernelement einen rechten Arm (364) und einen linken Arm (362) beinhaltet, die sich von dem Kernelement in entgegengesetzte seitliche Richtungen erstrecken, wobei jeder Arm einen zugeordneten Luftweg durch jeden jeweiligen Arm bildet, wobei jeder Arm eine Öffnung beinhaltet, die durch eine Seitenwand desselben bereitgestellt ist;
ein Paar von Nasenkissenanordnungen (100), wobei jede Nasenkissenanordnung mindestens ein Nasenkissen (110) aufweist, das drehbar über der Öffnung eines jeweiligen rechten Arms und linken Arms verbunden ist, wobei jede Nasenkissenanordnung dazu konfiguriert ist, die Zufuhr von mit Druck beaufschlagtem Gas von dem Luftweg durch jede Nasenkissenanordnung und zu den Nasenlöchern eines Benutzers zu kommunizieren;
eine Kopfbedeckungsschnittstelle (400), die um ein distales Ende jedes Arms gelegen ist, wobei die Kopfbedeckungsschnittstelle dazu konfiguriert ist, an der Kopfbedeckungsanordnung befestigt zu werden.

2. Maske und Kopfbedeckungsanordnung nach Anspruch 1, wobei jede Kopfbedeckungsschnittstelle ferner einen selektiv verformbaren Seitenbügel (500) umfasst, der dazu konfiguriert ist, an seinem jeweiligen Arm befestigt zu werden.

3. Maske und Kopfbedeckungsanordnung nach Anspruch 2, wobei der selektiv verformbare Seitenbügel dazu konfiguriert werden kann, in verschiedenen Winkelpositionen an dem Arm befestigt zu werden.

4. Maske und Kopfbedeckungsanordnung nach Anspruch 2, wobei der selektiv verformbare Seitenbügel flach und dazu konfiguriert ist, außerhalb der Ebene selektiv verformt zu werden, um etwa den Gesichtskonturen eines Benutzers zu entsprechen.

5. Maske und Kopfbedeckungsanordnung nach Anspruch 2, ferner umfassend:
eine formbare Hülse (510), die dazu konfiguriert ist, den selektiv verformbaren Seitenbügel zu umschließen.

6. Maske und Kopfbedeckungsanordnung nach Anspruch 1, ferner umfassend eine Befestigungshülse (150), die dazu konfiguriert ist, jeden von dem rechten und dem linken Arm jeweils in Eingriff zu nehmen und die zugeordnete Öffnung zu umschließen, wobei die Befestigungshülse dazu konfiguriert ist, eine Drehung jeder Nasenkissenanordnung um jeden jeweiligen Arm bereitzustellen, um einen Strom durch die jeweilige Öffnung aufrechtzuerhalten.

7. Maske und Kopfbedeckungsanordnung nach Anspruch 6, wobei die Befestigungshülse eine radiale Schlauchverbindung zum Bilden einer Schnittstelle mit einem jeweiligen Nasenkissen beinhaltet, wobei jedes Nasenkissen entlang des radialen Schlauchs axial einstellbar ist.

8. Maske und Kopfbedeckungsanordnung nach Anspruch 6, wobei jede Befestigungshülse mit einer oder mehreren Spüllöchern (158) bereitgestellt ist.

9. Maske und Kopfbedeckungsanordnung nach Anspruch 1, wobei ein Winkel, der zwischen dem rechten und linken Arm gebildet wird, weniger als 180 Grad beträgt.

10. Maske und Kopfbedeckungsanordnung nach Anspruch 1, wobei das Kernelement ferner Folgendes umfasst:
eines oder mehrere Spüllöcher (640), die um einen mittleren Abschnitt gelegen sind.

11. Maske und Kopfbedeckungsanordnung nach Anspruch 1, wobei das Kernelement ferner Folgendes umfasst:
eines oder mehrere Spüllöcher (159), die um die distalen Enden sowohl des rechten als auch des linken Arms gelegen sind.

12. Maske und Kopfbedeckungsanordnung nach Anspruch 1, wobei das Kernelement ferner Folgendes umfasst:
eine Wärme-Feuchtigkeitstauschvorrichtung (326), die um einen mittleren Abschnitt gelegen ist.

13. Maske und Kopfbedeckungsanordnung nach Anspruch 2, ferner umfassend eine flexible Abdeckung, die über einem Abschnitt von mindestens einem von den selektiv verformbaren Seitenbügeln der Kopfbedeckungsanordnung angeordnet ist.

14. Maske und Kopfbedeckungsanordnung nach Anspruch 1, wobei die Nasenkissenanordnung ferner ein Paar Befestigungshülse (150) umfasst, die zwischen jedem Arm des Kernelements und den Nasenkissen angeordnet sind, und wobei sich die Befestigungshülsen mindestens teilweise um jeden Arm drehen, während sie die Kommunikation des Stroms von mit Druck beaufschlagtem Gas von dem Luftweg durch die Nasenkissen aufrechterhält.

15. Maske und Kopfbedeckungsanordnung nach Anspruch 1, wobei der rechte und der linke Arm um das Kernelement schwenken.

## Revendications

1. Ensemble masque et harnais, le masque comprenant :
un noyau (300) ayant un raccord d'entrée (322) pour recevoir une alimentation en gaz sous pression provenant d'un tube de distribution, le noyau comportant un bras droit (364) et un bras gauche (362) s'étendant chacun depuis le noyau dans des directions latérales opposées, chaque bras formant un passage d'air associé à travers chaque bras respectif, dans lequel chaque bras comporte une ouverture prévue à travers une paroi latérale de celui-ci ;
une paire d'ensembles coussins nasaux (100), chaque ensemble coussin nasal ayant au moins un coussin nasal (110) relié de manière rotative sur l'ouverture d'un bras droit et d'un bras gauche respectifs, chaque ensemble coussin nasal étant configuré pour communiquer l'alimentation en gaz sous pression du passage d'air à travers chaque ensemble coussin nasal et jusqu'aux narines d'un utilisateur ;
une interface de harnais (400) située autour d'une extrémité distale de chaque bras, l'interface de harnais étant configurée pour être fixée à l'ensemble harnais.

2. Ensemble masque et harnais selon la revendication 1, dans lequel chaque interface de harnais comprend en outre une pièce latérale sélectivement déformable (500) configurée pour se fixer à son bras respectif.

3. Ensemble masque et harnais selon la revendication 2, dans lequel la pièce latérale sélectivement déformable peut être configurée pour se fixer au bras dans diverses positions angulaires.

4. Ensemble masque et harnais selon la revendication 2, dans lequel la pièce latérale sélectivement déformable est plane et configurée pour être sélectivement déformée hors du plan afin de se conformer aux contours du visage d'un utilisateur.

5. Ensemble masque et harnais selon la revendication 2, comprenant en outre :
un manchon malléable (510) configuré pour englober la pièce latérale sélectivement déformable.

6. Ensemble masque et harnais selon la revendication 1, comprenant en outre un manchon de fixation (150) configuré pour venir en prise avec chacun des bras droit et gauche, respectivement, et englober l'ouverture associée, le manchon de fixation étant configuré pour permettre la rotation de chaque ensemble coussin nasal autour de chaque bras respectif de manière à maintenir l'écoulement à travers l'ouverture respective.

7. Ensemble masque et harnais selon la revendication 6, dans lequel le manchon de fixation comporte un raccord de tuyau radial pour servir d'interface avec un coussin nasal respectif, dans lequel chaque coussin nasal est réglable axialement le long du tuyau radial.

8. Ensemble masque et harnais selon la revendication 6, dans lequel chaque manchon de fixation est muni d'un ou de plusieurs évents d'évacuation (158).

9. Ensemble masque et harnais selon la revendication 1, dans lequel un angle formé entre les bras droit et gauche est inférieur à 180 degrés.

10. Ensemble masque et harnais selon la revendication 1, dans lequel le noyau comprend en outre :
un ou plusieurs évents d'évacuation (640) situés autour d'une partie centrale.

11. Ensemble masque et harnais selon la revendication 1, dans lequel le noyau comprend en outre :
un ou plusieurs évents d'évacuation (159) situés autour des extrémités distales des bras droit et gauche.

12. Ensemble masque et harnais selon la revendication 1, dans lequel le noyau comprend en outre :
un dispositif d'échange d'humidité et de chaleur (326) situé autour d'une partie centrale.

13. Ensemble masque et harnais selon la revendication 2, comprenant en outre un couvercle flexible disposé sur une partie d'au moins l'une des pièces latérales sélectivement déformables de l'ensemble harnais.

14. Ensemble masque et harnais selon la revendication 1, dans lequel l'ensemble coussin nasal comprend en outre une paire de manchons de fixation (150) disposés entre chaque bras du noyau et les coussins nasaux, et dans lequel les manchons de fixation pivotent au moins partiellement autour de chaque bras tout en maintenant la communication du flux de gaz sous pression à partir du passage d'air jusqu'aux coussins nasaux.

15. Ensemble masque et harnais selon la revendication 1, dans lequel les bras droit et gauche pivotent autour du noyau.
